**Europäisches Patentamt**

**European Patent Office**

**Office européen des brevets**

(19)

(11) Veröffentlichungsnummer : **0 338 258 B1**

(12) # EUROPÄISCHE PATENTSCHRIFT

(45) Veröffentlichungstag der Patentschrift :
**29.07.92 Patentblatt 92/31**

(51) Int. Cl.⁵ : **A61K 6/04,** A61C 13/00,
**C22C 1/04, B22F 1/00**

(21) Anmeldenummer : **89104873.8**

(22) Anmeldetag : **18.03.89**

---

(54) **Verfahren zur Herstellung von gesinterten metallischen Zahnersatzteilen.**

---

(30) Priorität : **07.04.88 DE 3811628**

(43) Veröffentlichungstag der Anmeldung :
**25.10.89 Patentblatt 89/43**

(45) Bekanntmachung des Hinweises auf die
Patenterteilung :
**29.07.92 Patentblatt 92/31**

(84) Benannte Vertragsstaaten :
**AT BE CH DE ES FR GB GR IT LI LU NL SE**

(56) Entgegenhaltungen :
**DE-A- 3 027 472**
**DE-B- 2 828 304**

(56) Entgegenhaltungen :
**DE-B- 2 851 729**
**FR-A- 2 036 946**
**GB-A- 683 004**
**US-A- 4 261 745**

(73) Patentinhaber : **Heraeus Kulzer GmbH**
**Heraeusstr. 12 - 14**
**W-6450 Hanau (DE)**

(72) Erfinder : **Schebela, Heinz**
**Odenwaldstrasse 11**
**W-6369 Schöneck 1 (DE)**

(74) Vertreter : **Grimm, Ekkehard**
**Heraeus Holding GmbH Heraeusstrasse 12 -**
**14**
**W-6450 Hanau/Main (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von metallischen Zahnersatzteilen durch Aufbringen einer pastenförmigen Mischung aus Metallpulver und bei Temperaturerhöhung flüchtigem Bindemittel auf ein keramisches Modell, Erhitzen unter Verflüchtigen des Bindemittels und Sintern des Metallpulvers zu einem kompakten Metallkörper und Entfernen des keramischen Modells.

Aus der deutschen Patentschrift 714 164 ist ein Verfahren zum Herstellen von Zahnersatzteilen, wie Kronen, Brücken, Platten oder dergleichen, aus fein zerteiltem, schwer schmelzbarem Metall bekannt. Die fein zerteilten Metalle oder Metallmischungen werden mit plastisch machenden Stoffen, zum Beispiel Vaselin, angeteigt, auf ein Modell aus leicht zerstörbaren hitzebeständigen Werkstoffen aufgebracht und durch Erhitzen gesintert, worauf nach Entfernen des Modells die Zahnersatzteile durch Polieren usw. fertiggestellt werden.

Bei dem aus der deutschen Auslegeschrift 19 15 977 bekannten Verfahren zur Herstellung von gesintertem metallischem Zahnersatz werden pastenförmige Mischungen aus Metallpulver, dessen vor allem kugelförmige Teilchen nicht größer als 25 Mikrometer sind und vorzugsweise einen Durchmesser von etwa 2 - 15 Mikrometer besitzen, und bei Temperaturerhöhung flüchtigem Bindemittel als Ausgangsmaterial eingesetzt. Die pastenförmigen Mischungen werden auf das keramische Modell eines beschliffenen Zahnes aufgetragen und modelliert. Durch Erhitzen wird dann zunächst das Bindemittel entfernt und anschließend das Metallpulver zu einem kompakten Metallkörper gesintert. Um die Bildung von Hohlräumen in dem Metallkörper zu vermeiden, kann die sogenannte Flüssigphasen-Sinterung, wobei zwei oder mehr der in der Mischung vorhandenen pulverförmigen Metalle eine bei relativ niedriger Temperatur schmelzende eutektische Phase (Beispiel: 5 % Silicium, 95 % Gold; Schmelzpunkt 400 °C) bilden, oder zusätzlich ein Edelmetallresinat angewandt werden. Beispiele für das Metallpulver sind kugelförmiges Pulver aus Gold, Gold-Platin-Palladium-Silber-Legierung und Gold-Platin-Palladium-Silber-Gemisch, dem 2 - 5 % Silicium- oder Aluminiumpulver beigemischt werden; die Sintertemperatur beträgt etwa 1040 °C.

In der deutschen Offenlegungsschrift 35 32 331 wird ein ähnliches Verfahren der sintertechnologischen Herstellung eines metallischen Zahnersatzes, der mit Keramik oder Kunststoff verblendet werden kann, beschrieben. Dabei wird ein wässeriger Schlicker aus einem Gemisch von Metallpulvern mehrmodaler Größenverteilung mit groben und feinen Fraktionen und gegebenenfalls Glas- oder Keramik-Pulvern auf das als Brennträger dienende keramische Zahnmodell aufgetragen, modelliert und, um einen paßgenauen und hinreichend dichten Zahnersatz zu erhalten, bei einer Temperatur, die die Solidustemperatur mindestens eines Bestandteiles des Metallpulvergemisches übersteigt, gesintert. Vorzugsweise werden für die groben Fraktionen des Pulvergemisches, die eine Korngröße zwischen 30 und 100 Mikrometer aufweisen, überwiegend Pulver kugeliger und für die feinen Fraktionen, deren Korngröße unter 50 Mikrometer liegt, Pulver beliebiger Gestalt verwendet. Zur Veredlung der Oberfläche und zur Schließung von Poren kann auf die Oberfläche des gesinterten Schlickers ein niedrigschmelzendes Metall aufgesintert werden.

Das deutsche Gebrauchsmuster 87 10 561 betrifft mit Kunststoff oder Keramik verblendete Zahnersatzkörper, deren Metallgerüste durch Sintern einer Suspension vorzugsweise aus Edelmetallpulver, wie Platinpulver, Silberpulver, Palladiumpulver, Goldpulver und/oder Iridiumpulver, Gläsern, Oxiden, Nitriden, Carbiden, NE-Metallpulver, Lösemittel sowie Bindemittel erhalten werden. Zur Versinterung ist eine Temperatur bis zu 1500 °C anwendbar.

Das Sintern unter Bedingungen, die eine flüssige metallische Phase entstehen lassen, erleichtert die Erzeugung dichter metallischer Zahnersatzteile. Es hat sich jedoch gezeigt, daß unter solchen Bedingungen Zahnersatzteile mit abgerundeten Kanten, zum Beispiel Kronen mit unscharfem Rand, entstehen können. Auch darf die schmelzflüssige Phase nur in solchem Ausmaß auftreten, daß die Formstabilität des Sinterkörpers während des Sinterns erhalten bleibt.

Es stellt sich daher die Aufgabe der Erfindung, ein Verfahren der eingangs charakterisierten Art zum Sintern von pastenförmigen Mischungen aus Metallpulvern und Bindmitteln zu finden, mit dessen Hilfe sich ohne die Erzeugung schmelzflüssiger Phasen und ohne die zusätzliche Anwendung eines Edelmetallresinats beziehungsweise eines niedrigschmelzenden Metalls zur Oberflächenbehandlung dichte metallische Zahnersatzteile herstellen lassen. Die Zahnersatzteile sollen auch eine gute Gewebeverträglichkeit besitzen.

Die Aufgabe wird erfindungsgemäß dadurch gelöst, daß das Metallpulver aus Palladium und Gold und/oder Silber besteht und eine mittlere Teilchengröße von 0,5 - 1,5 Mikrometer besitzt und das Sintern unter Bildung einer Legierung aus Palladium und Gold und/oder Silber bei 900 - 950 °C erfolgt.

Bewährt hat sich das Metallpulver aus 15 - 75 Gewichts-% Palladium und 25 - 85 Gewichts-% Gold und/oder Silber.

Bevorzugte Pulver sind solche aus
a) 50 - 75 Gewichts-% Palladium und 25 - 50 Gewichts-% Silber und
b) 15 - 75 Gewichts-% Palladium, 15 - 50 Gewichts-% Gold und 10 - 35 Gewichts-% Silber.

2

Die Menge an Metallpulver in der pastenförmigen Mischung hängt von der Art des herzustellenden Zahnersatzteiles ab und beträgt im allgemeinen etwa 75 - 95 Gewichts-%, bezogen auf die Mischung.

Die das Metallpulver bildenden Teilchen können jede beliebige Form haben. Kugelförmige und/oder plättchenförmige Teilchen werden jedoch bevorzugt.

Für die Zubereitung der pastenförmigen Mischung können zum Beispiel die in der deutschen Auslegeschrift 19 15 977 und in der deutschen Patentschrift 28 51 729 beschriebenen Bindemittel benutzt werden.

Die nach dem Verfahren gemäß der Erfindung aus Metallpulvern durch Sintern hergestellten metallischen Zahnersatzteile sind homogen, dicht und auch an der Oberfläche porenfrei. Ein weiterer Vorteil des Verfahrens ist die relativ niedrige Sintertemperatur, bei der keine flüssige Phase entsteht, so daß die Zahnersatzteile scharf ausgebildete Ränder und Kanten aufweisen. Messerscharf auslaufende Kronenränder kennzeichnen nach dem Verfahren hergestellte Zahnkronen.

DE-A-30 27 472 betrifft ein Gold-Präparat zum Überziehen von metallischen Teilen, besonders von aus Aufbrennlegierungen hergestellten Kronen und Brücken, bevor diese mit einer Keramik-Verblendung versehen werden. Das Gold-Präparat enthält Gold-Pulver mit einer nach Anspruch 1 unterhalb von 30 Mikrometer, nach Anspruch 3 unterhalb von 10 Mikrometer liegenden Teilchengröße und daneben ein als Haftmittel wirkendes Metall-Pulver, dessen Teilchengröße nach Anspruch 1 größer als die des Gold-Pulvers ist. Das Metall-Pulver kann zum Biespiel ein Palladium-Pulver mit einer Teilchengröße unterhalb von 37 Mikrometer sein (Biespiel 3). Die aus dem Gold-Präparat durch Aufbrennen zwischen 600 und 1100°C auf den metallischen Teilen erzeugten Überzüge besitzen eine rauhe Oberfläche (Seite 5, letzter Absatz) und bewirken eine sehr gute Verankerung der Keramik-Verblendung (Seite 6, 2. Absatz).

Dagegen betrifft das vorliegende Patent ein Verfahren zur Herstellung von metallischen Zahnersatzteilen, also solcher Teile, die mit dem aus DE-A-30 27 472 bekannten Gold-Präparat überzogen werden können (siehe vorliegende Patentschrift, Anspruch 10). Für das Verfahren wird ein aus Palladium und Gold und/oder Silber bestehendes Pulver mit einer mittleren Teilchengröße von 0,5 - 1,5 Mikrometer benutzt, das bei 900 - 950°C gesintert wird. Die - im Vergleich zu DE-A-30 27 472 einheitliche Teilchengröße des Pulvers und die die Bildung einer flüssigen Phase ausschließende Sintertemperatur führen zu homogenen, dichten und auch an der Oberfläche porenfreien metallischen Zahnersatzteilen.

Soll nach dem erfindungsgemäßen Verfahren ein vollständig aus Metall bestehendes Zahnersatzteil, zum Beispiel eine Vollkrone, hergestellt werden, so wird auf das aus Keramik gefertigte Modell eines Zahnstumpfes die pastenförmige Mischung aus Metallpulver und Bindemittel schichtweise aufgetragen und anatomisch modelliert. Nach dem Trocknen wird das Modell samt den darauf aufgetragenen Pasten-Schichten in einen Ofen gesetzt und der Ofen langsam auf 900 - 950 °C aufgeheizt, wobei sich zunächst das Bindemittel verflüchtigt und dann das Metallpulver zu einem kompakten Metallkörper gesintert wird. Nach dem Abkühlen wird das Modell zerstört und so aus dem Metallkörper entfernt. Der Metallkörper besitzt eine völlig glatte und porenfreie Oberfläche; seine Kanten und Ränder sind scharf ausgeprägt.

Zur Herstellung von mit Keramik verblendetem Zahnersatz wird nach dem Sintern und Abkühlen Aufbrennkeramik, bestehend aus Grund-, Dentin- und Schmelzmasse, schichtweise auf den Metallkörper aufgetragen und gebrannt. Dann wird das Modell zerstört und der mit Keramik verblendete Zahnersatz ausgearbeitet.

Es ist auch möglich, das Modell schon nach dem Brand der Grundmasse-Schicht aus dem Metallkörper zu entfernen. Der nachfolgende anatomische Aufbau der Keramik-Verblendung kann dann auf dem aus Gips gefertigten Arbeitsmodell im Artikulator ausgeführt werden.

Vor dem Sintern des Metallkörpers können, um die Verbundfestigkeit zwischen Metallkörper und Aufbrennkeramik zu erhöhen, auf die letzte der auf das keramische Modell aufgetragenen Schichten der pastenförmigen Mischung Keramik-Teilchen, zum Beispiel Grundmasse-Pulver, aufgestreut werden.

Zur Herstellung von mit Kunststoff verblendetem Zahnersatz, zum Beispiel einer Metall/Kunststoff-Krone, wird nach dem Sintern, Abkühlen und Entfernen des Modells ein durch Polymerisation auszuhärtendes K+B-Material (Kronen- und Brückenmaterial) auf den Metallkörper aufgetragen und ausgehärtet.

Es hat sich bewährt, das keramische Modell, wie beispielsweise aus der deutschen Offenlegungsschrift 35 32 331 bekannt, vor dem Auftragen der pastenförmigen Mischung mit einer Isolierschicht, die das Eindringen des Bindemittels in das Modell verhindert, zu versehen.

Als besonders vorteilhaft hat sich eine Isolierschicht aus Aufbrennkeramik erwiesen. Das Entfernen des keramischen Modells aus dem gesinterten Metallkörper, zum Beispiel einer Kronenkappe, kann dann so vorgenommen werden, daß die aus Aufbrennkeramik bestehende Schicht ganz oder teilweise auf der Innenseite des Metallkörpers verbleibt. Die Aufbrennkeramik ist besonders gewebefreundlich und bewirkt in Verbindung mit dem Zement eine sehr gute Haftfestigkeit des Metallkörpers auf dem Zahnstumpf.

Das erfindungsgemäße Verfahren läßt sich vorteilhaft abwandeln, indem auf das keramische Modell zunächst pastenförmiges Blendgold, wie beispielsweise aus der deutschen Patentschrift 28 51 729 bekannt, aufgetragen, getrocknet und gesintert wird und dann eine ein Gemisch aus zum Beispiel 50 Gewichts-% Palladium-Pulver und 50 Gewichts-% Silber-Pulver enthaltende pastenförmige Mischung aufgetragen, getrocknet und zwischen etwa 900 und 950 °C gesintert wird.

Es ist ebenso möglich, nach dem Trocknen der Blendgold-Schicht zunächst die pastenförmige Mischung aufzutragen und dann Blendgold und Mischung gemeinsam bei einer Temperatur zwischen etwa 900 und 950 °C zu sintern.

Auch kann eine Blendgold-Schicht auf die pastenförmige Mischung aufgetragen und gemeinsam mit der pastenförmigen Mischung und gegebenenfalls der ersten Blendgold-Schicht gesintert oder auf den Metallkörper aufgetragen und gesintert werden. Falls gewünscht, können auf die noch nicht gesinterte äußere Blendgold-Schicht Keramik-Teilchen aufgestreut werden.

Durch die Mitverwendung von Blendgold bei dem Verfahren gemäß der Erfindung können zum Beispiel Kronenkappen hergestellt werden, deren mit dem Zahnstumpf und dem Zahnfleisch in Berührung kommende Innenflächen einen hohen Gold-Anteil aufweisen. Auch können so zum Beispiel Kronenkappen, zu deren Herstellung Palladium-Silber-Pasten benutzt werden, einen hochgoldhaltigen Überzug erhalten, der durch seinen gelben Farbton die ästhetische Wirkung der Keramik-Verblendungen, besonders solcher in heller Farbe und geringer Schichtdicke, erhöht.

Die folgenden Beispiele, in denen die Herstellung von Kronenkappen und ihre Verblendung mit Keramik beschrieben wird, erläutern das Verfahren.

## Beispiel 1

Von einem aus Gips bestehenden Arbeitsmodell eines Zahnstumpfes wird über eine Silicon-Dublierung ein keramisches Modell hergestellt, auf das eine Aufbrennkeramik-Schicht aufgebracht und gebrannt wird. Auf dem keramischen Modell wird mit der pastenförmigen Mischung aus 90 Gewichts-% eines Gemisches aus 35 Gewichts-% Palladium-Pulver, 30 Gewichts-% Gold-Pulver und 35 Gewichts-% Silber-Pulver mit einer mittleren Teilchengröße der Pulver von 0,5 - 1,5 Mikrometer und 10 Gewichts-% Äthylcellulose enthaltendem Terpineol schichtweise die Kronenkappe in grüner Form aufgebaut. Nach dem Trocknen werden die grüne Kronenkappe und das keramische Modell langsam auf 950 °C erhitzt, wobei sich das Bindemittel verflüchtigt und das Metallpulver zu einer kompakten, dichten und porenfreien Kronenkappe gesintert wird.

Auf die Kronenkappe wird keramische Grundmasse aufgetragen, verdichtet und bei etwa 960°C gebrannt. Nach dem Abkühlen wird dann das keramische Modell durch Ausstrahlen mit Glasperlen, wobei die Aufbrennkeramik-Schicht teilweise auf der Innenseite der Kronenkappe verbleibt, aus der Kronenkappe entfernt, die mit Grundmasse versehene Kronenkappe auf das Arbeitsmodell und beides zusammen in einen Artikular gesetzt. Der anatomische Aufbau mit Keramik und die Fertigstellung der mit Keramik verblendeten Kronenkappe erfolgen dann in bekannter Weise.

## Beispiel 2

Von einem aus Gips bestehenden Arbeitsmodell eines Zahnstumpfes wird über eine Silicon-Dublierung ein keramisches Modell hergestellt. Auf das Modell wird eine Schicht aus pastenförmigem Blendgold, wie in der deutschen Patentschrift 28 51 729 beschrieben, aufgetragen, getrocknet und bei 950°C gesintert. Dann wird die pastenförmige Mischung aus 95 Gewichts-% eines Gemisches aus 50 Gewichts-% Palladium-Pulver und 50 Gewichts-% Silber-Pulver mit einer mittleren Teilchengröße der Pulver von 0,5 - 1,5 Mikrometer und 5 Gewichts-% Äthylcellulose enthaltendem Terpineol schichtweise auf die gesinterte Blendgold-Schicht aufgetragen, getrocknet und bei 900°C ebenfalls gesintert. Nach dem Abkühlen wird nochmals pastenförmiges Blendgold aufgetragen, getrocknet und bei 820°C gesintert.

Es wird eine kompakte, dichte und porenfreie Kronenkappe, deren Oberfläche einen gelben Farbton aufweist, erhalten.

Auf die Kronenkappe wird keramische Grundmasse aufgetragen, verdichtet und gebrannt. Dann wird nach dem Abkühlen das keramische Modell im Ultraschallbad zerstört und, wie in Beispiel 1 beschrieben, eine mit Keramik verblendete Kronenkappe gefertigt.

## Patentansprüche

1. Verfahren zur Herstellung von metallischen Zahnersatzteilen durch Aufbringen einer pastenförmigen Mischung aus Metallpulver und bei Temperaturerhöhung flüchtigem Bindemittel auf ein keramisches Modell, Erhitzen unter Verflüchtigen des Bindemittels und Sintern des Metallpulvers zu einem kompakten Metallkörper und Entfernen des keramischen Modells, dadurch gekennzeichnet, daß das Metallpulver aus Palladium und Gold und/oder Silber besteht und eine mittlere Teilchengröße von 0,5 - 1,5 Mikrometer besitzt und das Sintern unter Bildung einer Legierung aus Palladium und Gold und/oder Silber bei 900 - 950 °C erfolgt.

2. Verfahren nach Anspruch 1, dadurch gekennzeichnet, daß das Metallpulver aus 15 - 75 Gewichts-% Palladium und 25 - 85 Gewichts-% Gold und/oder Silber besteht.

3. Verfahren nach Anspruch 2, dadurch gekennzeichnet, daß das Metallpulver aus 50 - 75 Gewichts-% Palladium und 25 - 50 Gewichts-% Silber besteht.

4. Verfahren nach Anspruch 2, dadurch gekennzeichnet, daß das Metallpulver aus 15 - 75 Gewichts-% Palladium, 15 - 50 Gewichts-% Gold und 10 - 35 Gewichts-% Silber besteht.

5. Verfahren nach einem der Ansprüche 1 bis 4, dadurch gekennzeichnet, daß die pastenförmige Mischung 75 - 95 Gewichts-% Metallpulver enthält.

6. Verfahren nach einem der Ansprüche 1 bis 5, dadurch gekennzeichnet, daß vor dem Aufbringen der pastenförmigen Mischung Blendgold auf das keramische Modell aufgetragen wird.

7. Verfahren nach Anspruch 6, dadurch gekennzeichnet, daß das Blendgold vor dem Aufbringen der pastenförmigen Mischung gesintert wird.

8. Verfahren nach Anspruch 7, dadurch gekennzeichnet, daß das Blendgold und die pastenförmige Mischung gleichzeitig gesintert werden.

9. Verfahren nach einem der Ansprüche 1 bis 8, dadurch gekennzeichnet, daß auf die pastenförmige Mischung Blendgold aufgetragen wird.

10. Verfahren nach einem der Ansprüche 1 bis 8, dadurch gekennzeichnet, daß Blendgold auf den Metallkörper aufgetragen und gesintert wird.

11. Verfahren nach einem der Ansprüche 1 bis 10, dadurch gekennzeichnet, daß auf das keramische Modell eine Isolierschicht aufgebracht wird.

12. Verfahren nach Anspruch 11, dadurch gekennzeichnet, daß die Isolierschicht durch Aufbringen und Brennen einer Aufbrennkeramik-Schicht erzeugt wird.

13. Verfahren nach Anspruch 12, dadurch gekennzeichnet, daß die Aufbrennkeramik-Schicht ganz oder teilweise auf der Innenseite des Metallkörpers verbleibt.

**Claims**

1. A method for the production of metallic tooth replacements through the application of a pasty mixture of metal powder and binding agent, which is volatile at an increase in temperature, onto a ceramic model, through heating, with volatilization of the binding agent and sintering of the metal powder to a compact metal body and removal of the ceramic model, characterised in that the metal powder consists of palladium and gold and/or silver and has a mean particle size of 0.5 - 1.5 micrometres and the sintering takes place with the formation of an alloy of palladium and gold and/or silver at 900 - 950 °C.

2. A method according to Claim 1, characterised in that the metal powder consists of 15 - 75 percentage by weight palladium and 25 - 85 percentage by weight gold and/or silver.

3. A method according to Claim 2, characterised in that the metal powder consists of 50 - 75 percentage by weight palladium and 25 - 50 percentage by weight silver.

4. A method according to Claim 2, characterised in that the metal powder consists of 15 - 75 percentage by weight palladium, 15 - 50 percentage by weight gold and 10 - 35 percentage by weight silver.

5. A method according to one of Claims 1 to 4, characterised in that the pasty mixture contains 75 - 95 percentage by weight metal powder.

6. A method according to one of Claims 1 to 5, characterised in that before the application of the pasty mixture, facing gold is applied onto the ceramic model.

7. A method according to Claim 6, characterised in that the facing gold is sintered before the application of the pasty mixture.

8. A method according to Claim 7, characterised in that the facing gold and the pasty mixture are sintered at the same time.

9. A method according to one of Claims 1 to 8, characterised in that facing gold is applied onto the pasty mixture.

10. A method according to one of Claims 1 to 8, characterised in that facing gold is applied onto the metal body and is sintered.

11. A method according to one of Claims 1 to 10, characterised in that an insulating layer is applied onto the ceramic model.

12. A method according to Claim 11, characterised in that the insulating layer is produced by the application and burning of a burnt-on ceramic layer.

13. A method according to Claim 12, characterised in that the burnt-on ceramic layer remains wholly or partly on the inner side of the metal body.

**Revendications**

1. Procédé de fabrication de prothèses dentaires métalliques par application d'un mélange pâteux d'une poudre métallique et d'un liant, volatilisable par élévation de la température, sur un modèle en céramique, chauffage avec volatilisation du liant et frittage de la poudre métallique en un corps métallique compact et enlèvement du modèle en céramique, caractérisé en ce que la poudre métallique se compose de palladium et d'or et/ou d'argent et possède une granulométrie moyenne comprise entre 0,5 et 1,5 μm et en ce que le frittage est opéré à 900-950°C avec formation d'un alliage de palladium et d'or et/ou d'argent.

2. Procédé selon la revendication 1, caractérisé en ce que la poudre métallique se compose de 15 à 75% en poids de palladium et de 25 à 85% en poids d'or et/ou d'argent.

3. Procédé selon la revendication 2, caractérisé en ce que la poudre métallique se compose de 50 à 75% en poids de palladium et de 25 à 50% en poids d'argent.

4. Procédé selon la revendication 2, caractérisé en ce que la poudre métallique se compose de 15 à 75% en poids de palladium, de 15 à 50% en poids d'or et de 10 à 35% en poids d'argent.

5. Procédé selon une des revendications 1 à 4, caractérisé en ce que le mélange pâteux contient 75 à 95% en poids de poudre métallique.

6. Procédé selon une des revendications 1 à 5, caractérisé en ce que de l'or de revêtement est déposé sur le modèle en céramique avant l'application du mélange pâteux.

7. Procédé selon la revendication 6, caractérisé en ce que l'or de revêtement est fritté avant l'application du mélange pâteux.

8. Procédé selon la revendication 7, caractérisé en ce que l'or de revêtement et le mélange pâteux sont frittés simultanément.

9. Procédé selon une des revendications 1 à 8, caractérisé en ce que de l'or de revêtement est déposé sur le mélange pâteux.

10. Procédé selon une des revendications 1 à 8, caractérisé en ce que de l'or de revêtement est déposé sur le corps métallique et fritté.

11. Procédé selon une des revendications 1 à 10, caractérisé en ce qu'une couche isolante est déposée sur le modèle en céramique.

12. Procédé selon la revendication 11, caractérisé en ce que la couche isolante est produite par application et cuisson d'une couche de céramique à cuire.

13. Procédé selon la revendication 12, caractérisé en ce que la couche de céramique à cuire demeure intégralement ou partiellement sur la face interne du corps métallique.